# EUROPEAN PATENT APPLICATION

(11) **EP 4 287 203 A1**
(43) Date of publication of application: **06.12.2023**
(21) Application number: 22178104.0
(22) Date of filing: 09.06.2022
(51) Int. Cl.: G16H 30/40, G16H 50/20, G16H 50/30

(54) **METHODS AND SYSTEMS FOR ANALYSIS OF LUNG ULTRASOUND**

(30) Priority: 01.06.2022 US 202263347615 P
(71) Applicant: Koninklijke Philips N.V., 5656 AG Eindhoven (NL)
(72) Inventor: KRUECKER, Jochen, Eindhoven (NL); CHEN, Alvin, Eindhoven (NL); BALARAJU, Naveen, 5656AG Eindhoven (NL)
(74) Representative: Philips Intellectual Property & Standards

(57) **Abstract**

A method (100) for analyzing ultrasound image data, comprising: (i) receiving (120) a temporal sequence of ultrasound image data for one or more of a plurality of different zones of one or both lungs of a patient; (ii) analyzing (130), using a first trained clinical lung feature identification algorithm, the received ultrasound image data to identify a first clinical feature in a lung of the patient, wherein identifying the first clinical feature comprises analysis of multiple frames in the temporal sequence, and wherein identifying the first clinical feature comprises identification of a location of the first clinical feature within the multiple frames; (iii) analyzing (140), using a trained clinical lung feature severity algorithm, the identified first clinical feature to characterize a severity of the identified first clinical feature; and (iv) providing (180), via a user interface, the identified first clinical feature and the characterized severity of the first clinical feature.

## Description

### FIELD OF THE INVENTION

The present disclosure is directed generally to methods and systems for analyzing lung ultrasound imaging to provide information about lung-related clinical features.

### BACKGROUND OF THE INVENTION

Lung ultrasound imaging is an important tool for disease screening, monitoring, diagnostic support, and management. Important clinical features - such as B-lines, merged B-lines, pleural line changes, consolidations, and pleural effusions, among others - can be identified using lung ultrasound. The presence of these features are predictors of a range of pulmonary and infectious diseases, including COVID-19 pneumonia. However, effectively identifying clinical features using lung ultrasound can depend on operator experience, image quality, and selection of imaging settings, among other variables. Thus, identifying clinical features is a challenging skill to learn, and success typically requires extensive specialized training and experience.

Automated quantification tools offer the potential to simplify and standardize image interpretation tasks, including ultrasound analysis. Studies have shown a correlation between automated lung ultrasound features and expert ratings, as well as correlation to gold standard measurements such as blood tests or chest CT. Automated analysis may even be used diagnostically for conditions such as COVID-19 pneumonia. Automated tools that utilize traditional image processing techniques are well-suited to extracting explainable image parameters that support human clinical interpretation. Image processing methods additionally benefit from potential advantages in simplicity, speed, and generalizability. A significant drawback, however, is that the performance of these techniques depends largely on the discriminatory power of the handcrafted parameters.

As an alternative to using handcrafted parameters derived from traditional image processing algorithms, machine learning and artificial intelligence-based techniques have gained popularity in the medical imaging domain, including for lung ultrasound applications.

While automated algorithms for detection and classification of lung ultrasound features are beneficial, review and reporting of features by a trained human operator are still needed. However, this human review can be time-consuming. For example, many clinical lung ultrasound features (e.g. B-lines, lung sliding, dynamic air bronchograms) require a dynamic view to assess the type or quality of the feature. Whole-video playback of the entire video loop is inefficient for review or reporting of individual features only present in part of the video, as this process requires either repeated playback of the full duration of the video in which only a part may be of interest to assess any particular feature, or repeated starting/stopping and replaying of a manually selected part of the video. Further, most lung ultrasound exams comprise multiple videos, each of which may require analysis. The reviewer must therefore manage the information from the plurality of videos that are part of an exam in order to determine the overall status of the patient.

### SUMMARY OF THE INVENTION

Accordingly, there is a need for automated lung ultrasound analysis tools capable of analyzing one or more lung ultrasound videos, also called cineloops, to identify lung-related clinical features.

The present disclosure is directed to inventive methods and systems for analysis of ultrasound lung imaging. Various embodiments and implementations herein are directed to an ultrasound analysis system optionally comprising an ultrasound device configured to obtain an ultrasound image of the patient's lungs. The system receives a temporal sequence of ultrasound image data for one or more of a plurality of different zones of one or both lungs of a patient. A first trained clinical lung feature identification algorithm analyzes the received temporal sequence of ultrasound image data to identify a first clinical feature in a lung of the patient, where identifying the first clinical feature comprises analysis of multiple frames in the temporal sequence, and where identifying the first clinical feature comprises identification of a location of the first clinical feature within the multiple frames. A trained clinical lung feature severity algorithm of the system analyzes the identified first clinical feature to characterize a severity of the identified first clinical feature. Optionally, a trained clinical feature prioritization algorithm analyzes the one or more identified clinical features to prioritize reporting of those features. A user interface of the system provides the identified first clinical feature and the characterized severity of the first clinical feature, and optionally provides the prioritization of the one or more identified clinical features.

Generally in one aspect, a method for analyzing ultrasound image data is provided. The method includes: (i) receiving a temporal sequence of ultrasound image data for one or more of a plurality of different zones of one or both lungs of a patient; (ii) analyzing, using a first trained clinical lung feature identification algorithm, the received temporal sequence of ultrasound image data to identify a first clinical feature in a lung of the patient, wherein identifying the first clinical feature comprises analyzing multiple frames in the temporal sequence, and wherein identifying the first clinical feature comprises identification of a location of the first clinical feature within the multiple frames; (iii) analyzing, using a trained clinical lung feature severity algorithm, the identified first clinical feature to characterize a severity of the identified first clinical feature; and (iv) providing, via a user interface, the identified first clinical feature and the characterized severity of the first clinical feature.

According to an embodiment, the method further includes analyzing, using the trained clinical lung feature identification algorithm, the received temporal sequence of ultrasound image data to identify a second clinical feature in a lung of the patient, wherein the second clinical feature is different from the first clinical feature; and analyzing, using the trained clinical lung feature severity algorithm, the identified second clinical feature to characterize a severity of the identified second clinical feature; wherein said providing step further comprises providing, via the user interface, the identified second clinical feature and the characterized severity of the second clinical feature.

According to an embodiment, the method further includes analyzing, using a second trained clinical lung feature identification algorithm, the received temporal sequence of ultrasound image data to identify a second clinical feature in a lung of the patient, wherein the second clinical feature is different from the first clinical feature; and analyzing, using a trained clinical lung feature severity algorithm, the identified second clinical feature to characterize a severity of the identified second clinical feature; wherein said providing step further comprises providing, via the user interface, the identified second clinical feature and the characterized severity of the second clinical feature.

According to an embodiment, the method further includes prioritizing, using a trained clinical feature prioritization algorithm, the identified first clinical feature or the identified second clinical feature, wherein prioritization is based on one or more of a type of the identified clinical feature, the characterized severity of the first clinical feature and second clinical feature, a timing of the first clinical feature and/or second clinical feature in the temporal sequence of ultrasound image data, and/or a suspected or diagnosed clinical condition of the patient; wherein said providing step further comprises providing said prioritization.

According to an embodiment, identifying a location of the first clinical feature within the multiple frames comprises identifying a spatiotemporal location across multiple frames.

According to an embodiment, providing the identified first clinical feature and the characterized severity of the first clinical feature comprises providing a subset of the received temporal sequence of ultrasound image data, the subset comprising the identified location of the identified first clinical feature. According to an embodiment, the subset is a temporal sequence. According to an embodiment, the subset is a static image.

According to an embodiment, the method further includes receiving, via the user interface, feedback from a user about the provided identified first clinical feature and/or the characterized severity of the first clinical feature. According to an embodiment, the feedback comprises an adjustment of the characterized severity of the first clinical feature, a selection of one or more frames in the temporal sequence of ultrasound image data, an acceptance or rejection of the feature, and/or a change of the type of feature.

According to an embodiment, the method further includes generating, based on the received feedback, a report comprising the identified first clinical feature and/or the characterized severity of the first clinical feature.

According to another aspect is an ultrasound analysis system configured to analyze ultrasound image data. The system includes: a temporal sequence of ultrasound image data for one or more of a plurality of different zones of one or both lungs of a patient; a trained clinical lung feature identification algorithm configured to analyze the received temporal sequence of ultrasound image data to identify a first clinical feature in a lung of the patient, wherein identifying the first clinical feature comprises analysis of multiple frames in the temporal sequence, and wherein identifying the first clinical feature comprises identification of a location of the first clinical feature within the multiple frames; a trained clinical lung feature severity algorithm configured to analyze the identified first clinical feature to characterize a severity of the identified first clinical feature; a processor configured to: (i) analyze, using the trained clinical lung feature identification algorithm, the received temporal sequence of ultrasound image data to identify a first clinical feature in a lung of the patient; (ii) analyze, using the trained clinical lung feature severity algorithm, the identified first clinical feature to characterize a severity of the identified first clinical feature; and a user interface configured to provide the identified first clinical feature and the characterized severity of the first clinical feature.

According to an embodiment, the system further comprises a trained clinical feature prioritization algorithm configured to prioritize one or more identified clinical features; the processor is further configured to prioritize, using the trained clinical feature prioritization algorithm one or more identified clinical features, wherein prioritization is based on one or more of a type of the identified clinical feature, the characterized severity of the first clinical feature and second clinical feature, a timing of the first clinical feature and/or second clinical feature in the temporal sequence of ultrasound image data, and/or a suspected or diagnosed clinical condition of the patient; and the user interface is further configured to provide said prioritization.

According to an embodiment, the processor is further configured to receive via the user interface, feedback from a user about the provided identified first clinical feature and/or the characterized severity of the first clinical feature. According to an embodiment, the processor is further configured to generate, based on the received feedback, a report comprising the identified first clinical feature and/or the characterized severity of the first clinical feature.

According to an embodiment, the user interface further comprises a summary display of the temporal sequence of ultrasound image data and the identified first clinical feature, wherein a user can select a region of the temporal sequence and/or the identified first clinical feature for review. According to an embodiment, after review by the user, the summary display of the temporal sequence of ultrasound image data and/or the identified first clinical feature is updated by the processor to show a status of the review.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 is a flowchart of a method for analyzing ultrasound image data using an ultrasound analysis system, in accordance with an embodiment.
Fig. 2 is a schematic representation of an ultrasound analysis system, in accordance with an embodiment.
Fig. 3 is a flowchart of a method for analyzing ultrasound image data using an ultrasound analysis system, in accordance with an embodiment.
Fig. 4 is a schematic representation of a graphical display of an ultrasound analysis system, in accordance with an embodiment.
Fig. 5 is a schematic representation of a graphical display of an ultrasound analysis system, in accordance with an embodiment.

### DETAILED DESCRIPTION OF EMBODIMENTS

The present disclosure describes various embodiments of an ultrasound analysis system and method. More generally, Applicant has recognized and appreciated that it would be beneficial to provide an ultrasound analysis that automatically generates information about lung-related clinical features. For example, an ultrasound analysis system receives or obtains ultrasound image data comprising lung-related clinical features. The system extracts and provides information about a plurality of different lung-related clinical features from the ultrasound image data.

The present disclosure describes various embodiments of an ultrasound analysis system and method. More generally, Applicant has recognized and appreciated that it would be beneficial to provide an ultrasound analysis that automatically generates about lung-related clinical features from temporal sequences of ultrasound image data. An ultrasound analysis system receives or obtains a temporal sequence of ultrasound image data for one or more of a plurality of different zones of one or both lungs of a patient. A first trained clinical lung feature identification algorithm analyzes the received temporal sequence of ultrasound image data to identify a first clinical feature in a lung of the patient, where identifying the first clinical feature comprises analysis of multiple frames in the temporal sequence, and wherein identifying the first clinical feature comprises identification of a location of the first clinical feature within the multiple frames. A trained clinical lung feature severity algorithm of the system analyzes the identified first clinical feature to characterize a severity of the identified first clinical feature. Optionally, a trained clinical feature prioritization algorithm analyzes the one or more identified clinical features to prioritize reporting of those features. A user interface of the system provides the identified first clinical feature and the characterized severity of the first clinical, and optionally provides the prioritization of the one or more identified clinical features.

According to an embodiment, the ultrasound analysis system and method disclosed or otherwise envisioned herein automatically identifies candidate features of interest in a lung ultrasound cineloop and - based on the feature type - provides a static or a feature-focused dynamic review of the feature. According to an embodiment, the reviewer can easily navigate between the candidate features without having to replay the entire video repeatedly, and without having to manually start/stop/restart the playback. According to an embodiment, by confirming or rejecting individual candidate features after review, a report on the lung ultrasound exam can be generated efficiently. Similarly, both during and after review, detected and/or confirmed features may be displayed in an efficient manner that doesn't require replaying the entire ultrasound video.

According to an embodiment, the systems and methods disclosed or otherwise envisioned herein can be used in any setting and on any system on which lung ultrasound is acquired and/or reviewed. In particular, the invention can be used on point-of-care and handheld ultrasound devices such as Philips Lumify^{®}, among many other devices and systems. The systems and methods could be used both as part of clinical practice and as a research and development tool to accelerate annotation workflows, among many other uses.

Thus, the ultrasound analysis system and method disclosed or otherwise envisioned herein provides numerous advantages over the prior art. Providing an ultrasound analysis system and method that enables the automated detection and analysis of lung-related clinical features in an understandable and interpretable manner can prevent serious lung injury, improve lung diagnoses and patient outcomes, and thus potentially save lives.

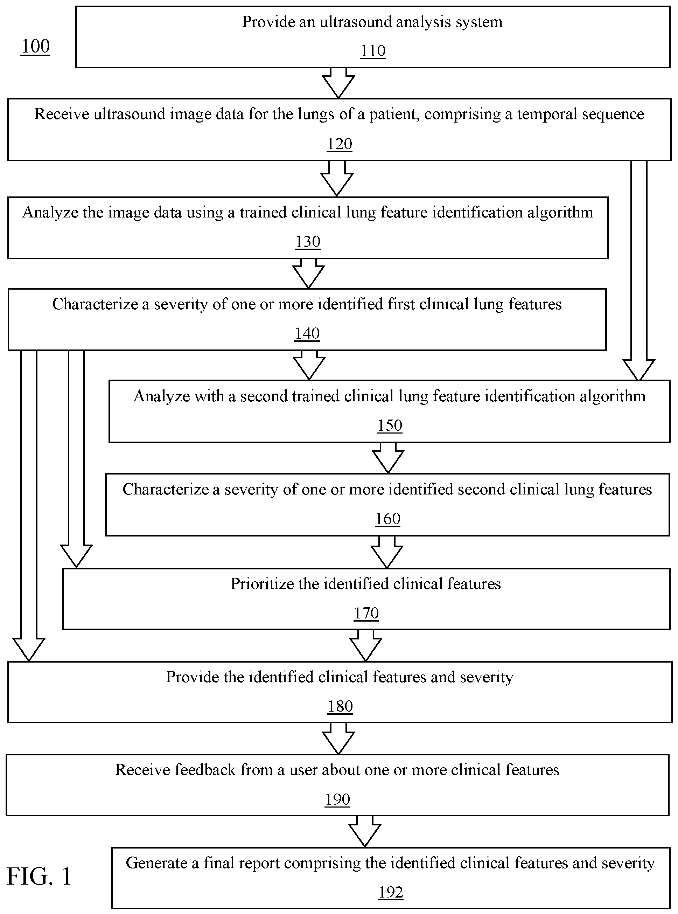

Referring to Fig. 1, in one embodiment, is a flowchart of a method 100 for analyzing ultrasound image data using an ultrasound analysis system. The methods described in connection with the figures are provided as examples only, and shall be understood not to limit the scope of the disclosure. The ultrasound analysis system can be any of the systems described or otherwise envisioned herein. The ultrasound analysis system can be a single system or multiple different systems.

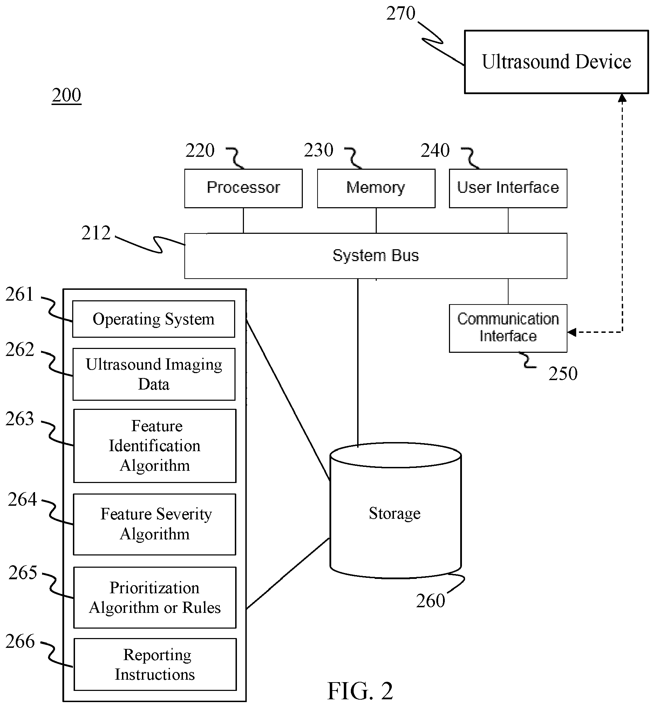

At step 110 of the method, an ultrasound analysis system 200 is provided. Referring to an embodiment of an ultrasound analysis system 200 as depicted in Fig. 2, for example, the system comprises one or more of a processor 220, memory 230, user interface 240, communications interface 250, storage 260, and ultrasound device 270, interconnected via one or more system buses 212. It will be understood that Fig. 2 constitutes, in some respects, an abstraction and that the actual organization of the components of the system 200 may be different and more complex than illustrated. Additionally, ultrasound analysis system 200 can be any of the systems described or otherwise envisioned herein. Other elements and components of system 200 are disclosed and/or envisioned elsewhere herein.

At step 120 of the method, ultrasound image data is sent to, obtained by, or otherwise received by the system. The ultrasound image data comprises a temporal sequence of ultrasound image data such as a video comprising a plurality of frames. Ultrasound image data may be obtained for a single region or zone of a patient's lung, or may be obtained for a plurality of different zones for one or more of the patient's lungs. For example, ultrasound image data may be obtained for one, two, or more zones. The ultrasound image data may be received by the system in real-time, or may be stored in local and/or remote memory and received by the system at a future point.

The ultrasound image data may be obtained using any ultrasound device or system, which may be any device or system suitable to obtain or otherwise receive ultrasound image data of the patient. One or more parameters of the ultrasound device can be set, adjusted, preprogrammed, or otherwise determined by a healthcare professional. The ultrasound device or system may be remote to, local to, or a component of, the ultrasound analysis system 200.

The ultrasound image data comprises data or other information about one or more of a plurality of different lung-related clinical features. According to an embodiment, a clinical feature is any recognizable aspect of a lung. A clinical feature may be a normal aspect of a lung or an abnormal aspect. A clinical feature may be indicative of a healthy lung or a diseased or injured lung. Thus, a clinical feature may be, for example, anything that can be identified within or from ultrasound image data. Examples of clinical features include A-lines, B-lines, merged B-lines, pleural line abnormalities, consolidation, pleural effusion, and many others.

At step 130 of the method, the ultrasound analysis system analyzes the received one or more temporal sequences of ultrasound image data to identify clinical features in the lung(s) of the patient. According to an embodiment, the ultrasound analysis system comprises a trained clinical lung feature identification algorithm that is configured to identify a clinical features in the lung(s) of the patient. Identifying the first clinical feature can comprise, for example, comparison of multiple frames in the temporal sequence. Identifying the first clinical feature can also comprise, for example, identification of a location of the first clinical feature within the multiple frames of the temporal sequence. Many other methods for identifying a clinical feature are possible. According to an embodiment, the trained clinical lung feature identification algorithm is configured to identify a specific type or types of clinical lung features. For example, the clinical lung feature identification algorithm can be trained to identify a specific type of clinical lung feature, such as A-lines, B-lines, merged B-lines, pleural line abnormalities, consolidation, or pleural effusion, among others.

As described above, according to one embodiment, the ultrasound analysis system comprises a single trained clinical lung feature identification algorithm configured to identify two or more different types of clinical feature in the lung(s) of the patient. According to another embodiment, the ultrasound analysis system comprises a plurality of trained clinical lung feature identification algorithms, each configured to identify one or more different types of clinical feature in the lung(s) of the patient. For example, a first clinical lung feature identification algorithm may be trained to identify or otherwise characterize B-lines. A second clinical lung feature identification algorithm may be trained to identify or otherwise characterize pleural line abnormalities, and so on.

The clinical lung feature identification algorithm(s) is trained, programmed, configured, or otherwise designed to specifically analyze a selected lung-related clinical feature, meaning that the trained algorithm will recognize and extract or identify information for the selected lung-related clinical feature. Thus, according to an embodiment, the ultrasound analysis system comprises one or more trained clinical lung feature identification algorithms, trained or configured to recognize and extract or identify one or more of the plurality of different possible lung-related clinical features. According to an embodiment, the clinical lung feature identification algorithm(s) or model(s) may be a deep neural network or may be another model such as random forest classifier, support vector machine classifier, boosting classifier, or any other type of machine learning model or algorithm. The clinical lung feature identification algorithm(s) may be trained using any method for training an algorithm or model, and may be stored in local and/or remote memory.

According to an embodiment, therefore, the ultrasound analysis system is configured to identify or otherwise characterize one or more lung ultrasound features of interest. The ultrasound analysis system is further configured with information about whether a feature should be reviewed or measured using a static ("S-type feature") or dynamic ("D-type feature") display. According to an embodiment, a preferred review mode - static or dynamic - is pre-defined for each clinical lung feature. For example, a static review mode may preferably be used for clinical lung features such as A-lines, pleural line abnormality, consolidation, atelectasis, and/or pleural effusion, among others. A dynamic review mode may preferably be used for clinical lung features such as B-lines, merged B-lines, dynamic air bronchogram, and lung sliding, among others.

The clinical lung feature identification algorithms of the ultrasound analysis system are configured or trained to identify spatiotemporal locations in the cineloop where the feature is likely present (i.e., "candidate features"). According to an embodiment, a clinical lung feature identification algorithm can utilize conventional image processing techniques including for example filtering, thresholding, spatial transformations and domain transformations such as Fourier transformations. The algorithm can also utilize machine learning techniques including Deep Learning and in particular convolutional neural networks (CNNs), trained to identify and detect and localize the feature. According to an embodiment, the algorithm is configured or trained to distinguish between different instances of the same feature type. For example, the same consolidation should only get counted once when selecting frames or short clips to review, and separate consolidations in a given video loop should get counted separately.

At step 140 of the method, the ultrasound analysis system analyzes or characterizes a severity of the identified clinical features identified by the analysis in step 130 of the method. According to an embodiment, the ultrasound analysis system comprises a trained clinical lung feature severity algorithm configured to characterize a severity of the identified first clinical feature. For example, the trained clinical lung feature severity algorithm can be configured to identify the severity of potentially multiple occurrences of a feature in a cineloop. According to an embodiment, the algorithm can utilize conventional image processing techniques or machine learning-based approaches, including CNNs, to determine the severity of a feature using multi-class classification or regression approaches. The algorithm may therefore utilize a variety of methods for the extraction of features and determination of feature severity.

At optional step 150 of the method, the ultrasound analysis system analyzes the received one or more temporal sequences of ultrasound image data to identify another type of clinical feature in the lung(s) of the patient. According to an embodiment, the ultrasound analysis system comprises a second, third, or more trained clinical lung feature identification algorithms each configured to identify a different type or variation of clinical feature in the lung(s) of the patient. As with the first algorithm, identifying a clinical feature can comprise, for example, analysis of multiple frames in the temporal sequence. Identifying a clinical feature can also comprise, for example, identification of a location of the clinical feature within the multiple frames of the temporal sequence. Many other methods for identifying the clinical feature are possible. According to an embodiment, the second trained clinical lung feature identification algorithm is configured to identify a specific type or types of clinical lung feature which is different from the type of clinical lung feature identified or otherwise analyzed by the first trained clinical lung feature identification algorithm.

Accordingly, at optional step 160 of the method, the ultrasound analysis system analyzes or characterizes a severity of the identified clinical features identified by the analysis in step 150 of the method. According to an embodiment, the ultrasound analysis system comprises a trained clinical lung feature severity algorithm configured to characterize a severity of the identified clinical feature. For example, the trained clinical lung feature severity algorithm can be configured to identify the severity of potentially multiple occurrences of a feature in a cineloop. According to an embodiment, the algorithm can utilize conventional image processing techniques or machine learning-based approaches, including CNNs, to determine the severity of a feature using multi-class classification or regression approaches. The algorithm may therefore utilize a variety of methods for the extraction of features and determination of feature severity. The trained clinical lung feature severity algorithm utilized in step 160 of the method may be the same algorithm utilized in step 140 of the method, or may be a separate or different trained clinical lung feature severity algorithm.

The order in which a plurality of trained clinical lung feature identification algorithms are utilized may depend on a variety of factors. According to an embodiment, the order of analysis by the system could be based on a user selection or option, predetermined programming, an aspect of the ultrasound exam itself such as the purpose for the exam or the type of exam, demographics or clinical information about the patient such as diagnosis, and/or other possible selection mechanisms. For example, a user could provide a list of one or more clinical features for analysis, or could select one or more clinical features from a menu of possible clinical features. As another option, the system could be configured, programmed, or otherwise designed to automatically analyze a given list of different clinical features, in a particular order or configuration. This automatic order or configuration, however, could be adjustable based on user input or other information such as the purpose for the exam or the type of exam, among many other possible adjustment mechanisms. According to yet another embodiment, the plurality of trained clinical lung feature identification algorithms may analyze the received ultrasound image data simultaneously.

At optional step 170 of the method, the ultrasound analysis system prioritizes the identified clinical features. According to an embodiment, the ultrasound analysis system comprises a clinical feature prioritization algorithm configured or trained to rank, prioritize, or otherwise sort or process the clinical lung features identified by the analyses of the lung ultrasound image data. According to an embodiment, the clinical feature prioritization algorithm configured or trained to determine which of the identified clinical lung features should be highlighted or provided to a user, and/or in what order the identified clinical lung features should be highlighted or provided to a user.

According to an embodiment, the clinical feature prioritization algorithm is trained or configured to determine an efficient order in which to display one or more detected candidate features to the user. The prioritization can be based on one or more of a type of the identified clinical feature, the characterized severity of the first clinical feature and second clinical feature, a timing of the first clinical feature and/or second clinical feature in the temporal sequence of ultrasound image data, and/or a suspected or diagnosed clinical condition of the patient, among many other options.

For example, prioritization can be based on the temporal occurrence of the identified feature in the cineloop, with early occurrence being prioritized over late occurrence, or vice versa. As another example, prioritization can be based on a fixed sequence of feature types, which can be programmed or determined by a user. For example, the fixed sequence can be: (1) B-lines (if any); (2) lung consolidations (if any); (3) pleural effusions (if any); (4) pleural line abnormalities (if any); (4) lung sliding abnormalities (if any); and/or (5) other features (if any). Many other orders are possible.

As another example, prioritization can be based on a suspected diagnosis, prognosis, and/or clinical condition of a patient. For example, for a patient suspected of COVID-19, the prioritization may be: 1. B-lines, 2. Pleural line abnormalities, 3. Consolidation, 4. Pleural effusion, and 5. Lung sliding, among other possible prioritizations. As another example, for a patient suspected of community-acquired pneumonia, the prioritization may be: 1. Consolidation, 2. B-lines, 3. Pleural line abnormalities, 4. Pleural effusion, and 5. Lung sliding, among other possible prioritizations. As another example, for a patient suspected of cardiogenic pulmonary edema, the prioritization may be: 1. B-lines, 2. Consolidation, 3. Pleural line abnormalities, 4. Pleural effusion, and 5. Other features, among other possible prioritizations. According to an embodiment, prioritization can be based on potential difficulty as determined by confidence scores. For example, priority may be given to easier candidates first and challenging candidates last. Many other prioritizations are possible.

At step 180 of the method, the ultrasound analysis system provides the identified clinical features and characterized severity of the clinical features to a user via a user interface of the system. Other information is possible as well, including but not limited to the identity of the patient, patient demographics, diagnosis or treatment information, and a wide variety of other possible information. The information can be provided via the user interface using any method for conveying or displaying information, and the user interface can be any device, interface, or mechanism for providing the conveyed or displayed information. The user can be any user reviewing lung ultrasound image data, including but not limited to a technician, medical professional, clinician, patient, and/or any other user.

In an embodiment in which several trained clinical lung feature identification algorithms analyze the data and identify different clinical features of different types, the user interface provides the different clinical features and the characterized severity of the different clinical features. In an embodiment in which a trained clinical feature prioritization algorithm prioritizes the clinical features, the user interface provides the prioritization information.

According to an embodiment, providing the identified clinical features to a user via a user interface of the system comprises providing a subset of the received temporal sequence of ultrasound image data. The subset can comprise, for example, the identified location of the identified first clinical feature. According to an embodiment, the subset is a temporal sequence less than a full temporal sequence received by the lung ultrasound system. According to an embodiment, the subset is one or more static images.

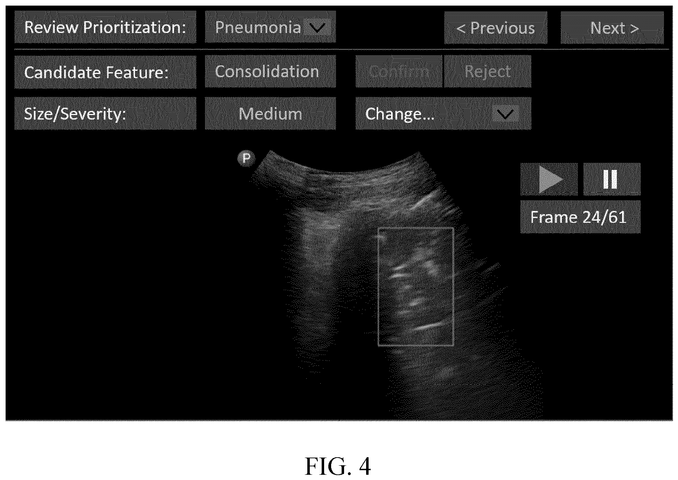

According to an embodiment, depending on the displayed feature type, the image review can be displayed dynamically with a playback of image sequences around the temporal center of the feature detection, or statically with a display of one or more frames in which feature is detected with highest confidence or is largest in size. According to an embodiment, the user interface can comprise a UI option to switch between static/dynamic view, such as a play/stop option. Referring to Fig. 4, in one embodiment, is an example of a user interface for review of a lung ultrasound cineloop.

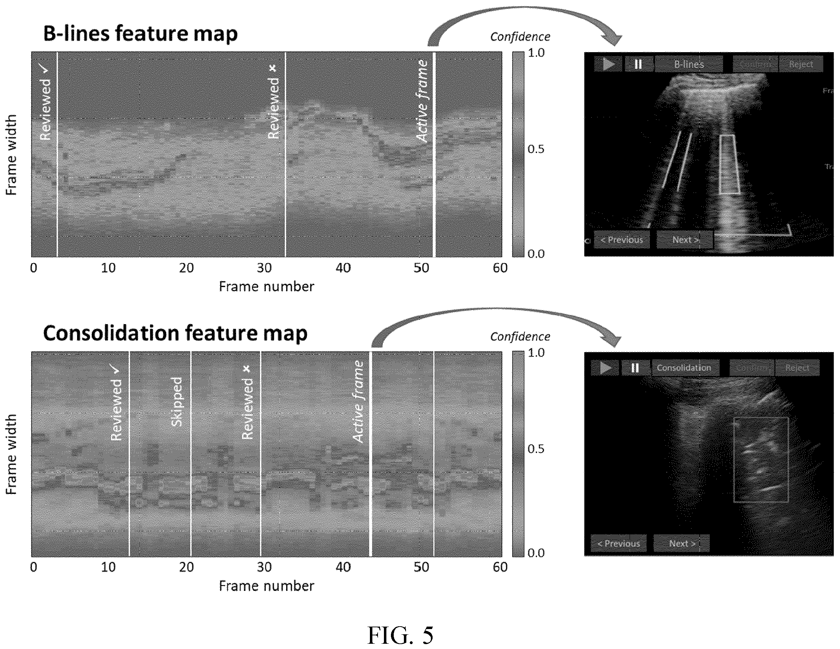

According to an embodiment, the user interface may also include a navigation pane to help the user move from one feature to the next during a review, and also to provide a snapshot of one or more detected features and their position along the video loop. Referring to Fig. 5, in one embodiment, is an example of how a navigation plane could be displayed, where frames are shown as a series of slices stacked together to form a heat map. The heat map could be an axial projection or any other format enabling compression of video data. For each feature, high confidence frames are highlighted, and the candidates detected from two or more are displayed. After review of each selected frame or short clip, the status of the review can be shown. For example, as shown in Fig. 5, the display has been updated to show review statuses such as "Reviewed," "Skipped," and "Active Frame." Although these labels are provided, they are non-limiting examples and many other labels - including words, graphics, and/or other indicators - could be utilized. Alternatively, rather than a separate map for each feature, there could be a single map showing all feature candidates where different feature types are displayed with different colors to easily tell them apart during review. The user can show/hide feature types depending on prioritization.

At optional step 190 of the method, a user provides feedback about the provided information via a user interface of the lung ultrasound analysis system. The user interface allows the user to review the provided information, and further enables the user to provide feedback to the system about that provided information. The feedback can be provided via the user interface using any method for conveying or providing information, and the user interface can be any device, interface, or mechanism for receiving the conveyed or provided information.

For example, the user can select or change the feature review prioritization provided or determined by the system, the user can select a cineloop for review from all the cineloops in an exam, the user can select to display a candidate feature type and/or severity, the user can select to display the part of the cineloop where the feature is present, either in dynamic or static view, the user can navigate to a next/previous candidate feature, the user can accept or reject or change a feature type and/or severity, the user can adjust the ROI around a detected feature (where, for example, manual adjustment in any frame around the candidate could result in a corresponding automatic adjustment of ROIs in all surrounding frames), and/or the user could add notes to be associated with a feature, among many other types of feedback.

At step 192 of the method, the lung ultrasound system generates a report comprising the identified first clinical feature and/or the characterized severity of the first clinical feature. Other information is possible as well, including but not limited to the identity of the patient, patient demographics, diagnosis or treatment information, and a wide variety of other possible information. The report can be provided via the user interface using any method for conveying or displaying information, and the user interface can be any device, interface, or mechanism for providing the conveyed or displayed information.

According to an embodiment, generation of the report may comprise a selection of one or several cineloops from an exam (where, for example, there may have been a selection of a name/number to differentiate selected cineloops, e.g. using lung zone numbering or naming, such as "Left Anterior Inferior (LAI)," etc.). Generation of the report may further comprise the display of sample images of each reviewed/confirmed feature in each cineloop of an exam, an option to include or exclude any of the images or features from the summary report, and/or a summary of the total number of features or the total number of frames containing each feature type. Many other features of a report are possible.

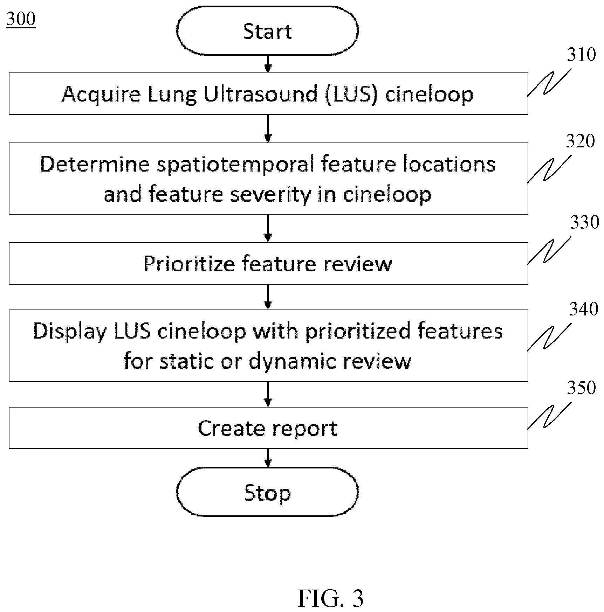

Referring to Fig. 3, in one embodiment, is a flowchart of a method 300 for generating a lung ultrasound feature report. At step 310, the system obtains, receives, or otherwise acquires one or more lung ultrasound (LUS) cineloops for one or more zones or regions of a patient's lungs. At step 320, the system utilizes one or more trained clinical lung feature identification algorithms to identify one or more clinical lung features, including spatiotemporal location(s) of the features. The system also utilizes one or more trained clinical lung feature severity algorithms to determine a severity of the identified one or more clinical lung features. At step 330, the system optionally prioritizes the identified one or more clinical lung features using a trained prioritization algorithm. At step 340, the system provides the one or more clinical lung features, the severity of the one or more clinical lung features, and/or the prioritization of the one or more clinical lung features via a user interface, using a static and/or dynamic review. At step 350 the system generates a report comprising the determined information, which may optionally include feedback received from the user.

Accordingly, the methods and systems described or otherwise envisioned herein provide numerous advantages over the prior art. For example, the system provides improved interpretability of ultrasound imaging compared to prior art systems, as a clinician is better able to evaluate more lung ultrasound imaging data, including more clinical lung features, in a shorter period of time. Detecting and visualizing relevant clinical lung features, and providing intermediate results at the frame/video/exam level, allows the user to interpret the ultrasound findings alongside other patient medical information and make a final, more-informed clinical judgment, thereby improving patient outcomes.

According to an embodiment, the methods and systems described or otherwise envisioned herein comprise numerous applications. For example, the system could be utilized in a pre-hospital setting, as an initial evaluation in an emergency room, for follow-up after a treatment, and in many other settings. The method is applicable to all ultrasound imaging systems, including in point-of-care applications. The methods and systems can be used in a variety of settings including ambulance, ER, or critical care, or surgery situations.

Referring to Fig. 2 is a schematic representation of an ultrasound analysis system 200. System 200 may be any of the systems described or otherwise envisioned herein, and may comprise any of the components described or otherwise envisioned herein. It will be understood that Fig. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may be located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, storage 260 of system 200 may store one or more algorithms, modules, and/or instructions to carry out one or more functions or steps of the methods described or otherwise envisioned herein. For example, the system may comprise, among other instructions or data, ultrasound imaging data 262, trained clinical lung feature identification algorithm 263, trained clinical lung feature severity algorithm 264, trained clinical feature prioritization algorithm 265, and/or reporting instructions 266, among many other possible instructions and/or data.

According to an embodiment, ultrasound imaging data 262 is any ultrasound imaging data that is sent to, obtained by, or otherwise received by the system. The ultrasound image data comprises a temporal sequence of ultrasound image data such as a video comprising a plurality of frames. Ultrasound image data may be obtained for a single region or zone of a patient's lung, or may be obtained for a plurality of different zones for one or more of the patient's lungs. For example, ultrasound image data may be obtained for one, two, or more zones. The ultrasound image data may be received by the system in real-time, or may be stored in local and/or remote memory and received by the system at a future point. The ultrasound image data may be obtained using any ultrasound device or system 270, which may be any device or system suitable to obtain or otherwise receive ultrasound image data of the patient. The ultrasound device or system may be remote to, local to, or a component of, the ultrasound analysis system 200.

According to an embodiment, trained clinical lung feature identification algorithm 263 is any model or algorithm that is trained or configured to analyze the received ultrasound image data to identify a clinical features in the lung(s) of the patient. Identifying the first clinical feature can comprise, for example, analysis of multiple frames in the temporal sequence. Identifying the first clinical feature can also comprise, for example, identification of a location of the first clinical feature within the multiple frames of the temporal sequence. According to an embodiment, the trained clinical lung feature identification algorithm is configured to identify a specific type or types of clinical lung features. For example, the clinical lung feature identification algorithm can be trained to identify a specific type of clinical lung feature, such as A-lines, B-lines, merged B-lines, pleural line abnormalities, consolidation, or pleural effusion, among others. According to an alternative embodiment, the ultrasound analysis system comprises a plurality of trained clinical lung feature identification algorithms, each configured to identify one or more different types of clinical features in the lung(s) of the patient. For example, a first clinical lung feature identification algorithm may be trained to identify or otherwise characterize B-lines. A second clinical lung feature identification algorithm may be trained to identify or otherwise characterize pleural line abnormalities, and so on. According to an embodiment, the clinical lung feature identification algorithm(s) or model(s) may be a deep neural network or may be another model such as random forest classifier, support vector machine classifier, boosting classifier, or any other type of machine learning model or algorithm. The clinical lung feature identification algorithm(s) may be trained using any method for training an algorithm or model.

According to an embodiment, trained clinical lung feature severity algorithm 264 is any model or algorithm that is trained or configured to analyze the received ultrasound image data to characterize a severity of the identified first clinical feature. For example, the trained clinical lung feature severity algorithm can be configured to identify the severity of potentially multiple occurrences of a feature in a cineloop. According to an embodiment, the algorithm can utilize conventional image processing techniques or machine learning-based approaches, including CNNs, to determine the severity of a feature using multi-class classification or regression approaches. The algorithm may therefore utilize a variety of methods for the extraction of features and determination of feature severity.

According to an embodiment, trained clinical feature prioritization algorithm 265 is any model, algorithm, or set of rules that is utilized, trained, or configured to analyze the identified clinical features in order to rank, prioritize, or otherwise sort or process the clinical lung features identified by the analyses of the lung ultrasound image data. According to an embodiment, the clinical feature prioritization algorithm configured or trained to determine which of the identified clinical lung features should be highlighted or provided to a user, and/or in what order the identified clinical lung features should be highlighted or provided to a user. According to an embodiment, the clinical feature prioritization algorithm is trained or configured to determine an efficient order in which to display one or more detected candidate features to the user. The prioritization can be based on one or more of a type of the identified clinical feature, the characterized severity of the first clinical feature and second clinical feature, a timing of the first clinical feature and/or second clinical feature in the temporal sequence of ultrasound image data, and/or a suspected or diagnosed clinical condition of the patient, among many other options.

According to an embodiment, reporting instructions 266 direct the system to generate and provide a report or visualization to a user via the user interface 240 of the ultrasound analysis system 200. The report or visualization comprises, for example, any information generated by the system during the analysis, including but not limited to the identified clinical features and characterized severity of the clinical features to a user via a user interface of the system. Other information is possible as well, including but not limited to the identity of the patient, patient demographics, diagnosis or treatment information, and a wide variety of other possible information. The information can be provided via the user interface using any method for conveying or displaying information, and the user interface can be any device, interface, or mechanism for providing the conveyed or displayed information. The user can be any user reviewing lung ultrasound image data, including but not limited to a technician, medical professional, clinician, patient, and/or any other user.

According to an embodiment, reporting instructions 266 direct the system to generate and provide a final report that may include, for example, feedback from a user. The final report may include the identified clinical features and/or the characterized severity of the clinical features. Other information is possible as well, including but not limited to the identity of the patient, patient demographics, diagnosis or treatment information, and a wide variety of other possible information. The report can be provided via the user interface using any method for conveying or displaying information, and the user interface can be any device, interface, or mechanism for providing the conveyed or displayed information. According to an embodiment, generation of the report may comprise a selection of one or several cineloops from an exam (where, for example, there may have been a selection of a name/number to differentiate selected cineloops, e.g. using lung zone numbering or naming, such as "Left Anterior Inferior (LAI)," etc.). Generation of the report may further comprise the display of sample images of each reviewed/confirmed feature in each cineloop of an exam, an option to include or exclude any of the images or features from the summary report, and/or a summary of the total number of features or the total number of frames containing each feature type. Many other features of a report are possible.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for analyzing ultrasound image data, comprising:
receiving (120) a temporal sequence of ultrasound image data for one or more of a plurality of different zones of one or both lungs of a patient;
analyzing (130), using a trained clinical lung feature identification algorithm, the received temporal sequence of ultrasound image data to identify a first clinical feature in a lung of the patient, wherein identifying the first clinical feature comprises analysis of multiple frames in the temporal sequence, and wherein identifying the first clinical feature comprises identification of a location of the first clinical feature within the multiple frames;
analyzing (140), using a trained clinical lung feature severity algorithm, the identified first clinical feature to characterize a severity of the identified first clinical feature; and
providing (180), via a user interface, the identified first clinical feature and the characterized severity of the first clinical feature.

2. The method of claim 1, further comprising the steps of:
analyzing (150), using the trained clinical lung feature identification algorithm, the received temporal sequence of ultrasound image data to identify a second clinical feature in a lung of the patient, wherein the second clinical feature is different from the first clinical feature; and
analyzing (160), using the trained clinical lung feature severity algorithm, the identified second clinical feature to characterize a severity of the identified second clinical feature;
wherein said providing step further comprises providing, via the user interface, the identified second clinical feature and the characterized severity of the second clinical feature.

3. The method of claim 1, further comprising the steps of:
analyzing (150), using a second trained clinical lung feature identification algorithm, the received temporal sequence of ultrasound image data to identify a second clinical feature in a lung of the patient, wherein the second clinical feature is different from the first clinical feature; and
analyzing (160), using the trained clinical lung feature severity algorithm, the identified second clinical feature to characterize a severity of the identified second clinical feature;
wherein said providing step further comprises providing, via the user interface, the identified second clinical feature and the characterized severity of the second clinical feature.

4. The method of claim 1, further comprising:
prioritizing (170), using a trained clinical feature prioritization algorithm, the identified first clinical feature or the identified second clinical feature, wherein prioritization is based on one or more of a type of the identified clinical feature, the characterized severity of the first clinical feature and second clinical feature, a timing of the first clinical feature and/or second clinical feature in the temporal sequence of ultrasound image data, and/or a suspected or diagnosed clinical condition of the patient;
wherein said providing step further comprises providing said prioritization.

5. The method of claim 1, wherein identifying a location of the first clinical feature within the multiple frames comprises identifying a spatiotemporal location across multiple frames.

6. The method of claim 1, wherein providing the identified first clinical feature and the characterized severity of the first clinical feature comprises providing a subset of the received temporal sequence of ultrasound image data, the subset comprising the identified location of the identified first clinical feature.

7. The method of claim 5, wherein the subset is a temporal sequence.

8. The method of claim 1, further comprising:
receiving (190), via the user interface, feedback from a user about the provided identified first clinical feature and/or the characterized severity of the first clinical feature.

9. The method of claim 8, wherein the feedback comprises an adjustment of the characterized severity of the first clinical feature, a selection of one or more frames in the temporal sequence of ultrasound image data, an acceptance or rejection of the feature, and/or a change of the type of feature.

10. An ultrasound analysis system (200) configured to analyze ultrasound image data, comprising:
a temporal sequence of ultrasound image data (262) for one or more of a plurality of different zones of one or both lungs of a patient;
a trained clinical lung feature identification algorithm (263) configured to analyze the received temporal sequence of ultrasound image data to identify a first clinical feature in a lung of the patient, wherein identifying the first clinical feature comprises analysis of multiple frames in the temporal sequence, and wherein identifying the first clinical feature comprises identification of a location of the first clinical feature within the multiple frames;
a trained clinical lung feature severity algorithm (264) configured to analyze the identified first clinical feature to characterize a severity of the identified first clinical feature;
a processor (220) configured to: (i) analyze, using the trained clinical lung feature identification algorithm, the received temporal sequence of ultrasound image data to identify a first clinical feature in a lung of the patient; (ii) analyze, using the trained clinical lung feature severity algorithm, the identified first clinical feature to characterize a severity of the identified first clinical feature; and
a user interface (240) configured to provide the identified first clinical feature and the characterized severity of the first clinical feature.

11. The ultrasound analysis system of claim 10, wherein:
the system further comprises a trained clinical feature prioritization algorithm (265) configured to prioritize one or more identified clinical features;
the processor is further configured to prioritize, using the trained clinical feature prioritization algorithm one or more identified clinical features, wherein prioritization is based on one or more of a type of the identified clinical feature, the characterized severity of the first clinical feature and second clinical feature, a timing of the first clinical feature and/or second clinical feature in the temporal sequence of ultrasound image data, and/or a suspected or diagnosed clinical condition of the patient; and
the user interface is further configured to provide said prioritization.

12. The ultrasound analysis system of claim 10, wherein providing the identified first clinical feature and the characterized severity of the first clinical feature comprises providing a subset of the received temporal sequence of ultrasound image data, the subset comprising the identified location of the identified first clinical feature.

13. The ultrasound analysis system of claim 10, wherein the processor is further configured to:
receive via the user interface, feedback from a user about the provided identified first clinical feature and/or the characterized severity of the first clinical feature; and
generate, based on the received feedback, a report comprising the identified first clinical feature and/or the characterized severity of the first clinical feature.

14. The ultrasound analysis system of claim 10, wherein the user interface further comprises a summary display of the temporal sequence of ultrasound image data and the identified first clinical feature, wherein a user can select a region of the temporal sequence and/or the identified first clinical feature for review.

15. The ultrasound analysis system of claim 14, wherein, after review by the user, the summary display of the temporal sequence of ultrasound image data and/or the identified first clinical feature is updated by the processor to show a status of the review.
